# EUROPEAN PATENT APPLICATION

(11) **EP 0 523 529 A2**
(43) Date of publication of application: **20.01.1993**
(21) Application number: 92111592.9
(22) Date of filing: 08.07.1992
(51) Int. Cl.: A61K 31/635

(54) **Anticoccidial compositions**

(30) Priority: 17.07.1991 US 731682
(71) Applicant: F. HOFFMANN-LA ROCHE AG, CH-4002 Basel (CH)
(72) Inventor: Schildknecht, Eugene George, Hackettstown, NJ 07840 (US); Untawale, Govind Gajanan, Wayne, NJ 07470 (US)
(74) Representative: Cottong, Norbert A.

(57) **Abstract**

Compositions for combating coccidiosis in animals comprise a mixture of the antibiotic frenolicin B with a potentiated sulfonamide anticoccidial agent, namely sulfadimethoxine potentiated with ormetoprim. Such compositions can be incorporated in animal feed or drinking water to afford anticoccidial animal comestible compositions and animal drinking water compositions respectively, with which the present invention is also concerned. Animal feed additive premixes containing the aforementioned frenolicin B/potentiated sulfonamide anticoccidial compositions and useful for producing the medicated animal feeds represent a further aspect of the invention.

## Description

The invention relates to the field of compositions useful for the prevention and treatment of coccidiosis in animals, methods of using such compositions and animal comestibles containing such compositions.

Coccidiosis is a disease caused by microscopic protozoal parasites called coccidia, belonging to the genus Eimeria. The infection in the host animals is initiated by the ingestion, usually along with food, water, and/or fecal material, of Eimeria organisms in the sporulated oocyst stage. When the ingested oocysts enter the intestine, the infectious stage of the Eimeria soon develops from the oocysts and causes extensive damage to the inner walls of the intestine and the cecum or "intestinal pouch." Cecal coccidiosis in chickens, for example, is caused primarily by the organism E. tenella and results in the destruction of the cecal linings of the host.

A number of coccidiostatic agents are presently available for the prevention and/or treatment of coccidiosis. Still, many of these agents have certain shortcomings. Animals treated with some of the known coccidiostats sometimes show reduced feed efficiency and lower weight gains than normal. Moreover, the development of resistance to the more commonly used agents is becoming an increasingly significant problem, one which is becoming a limiting factor in successfully combating coccidiosis. Still other agents have very narrow safety and efficacy ranges with resulting toxicity risks for treated animals, as well as for other farm animals and man by virtue of incidental or accidental exposure or ingestion.

A naphthoquinone antibiotic produced from Streptomyces roseofulvus, frenolicin B, has been shown to have some anticoccidial activity against E. tenella in chickens (Omura, et al., J. Antibiotics, Vol. 38, No. 10, pp. 1447-8 (1985)). Frenolicin B has recently been shown, e.g. in U.S. Patent Specification No. 4,839,382, to be synergistic in combination with ionophoric anticoccidial antibiotics against which Eimeria field isolates had developed resistance due to the chronic usage of these agents to combat coccidiosis.

It has now been discovered that compositions that contain a combination of frenolicin B and a potentiated sulfonamide anticoccidial, namely sulfadimethoxine potentiated with ormetoprim, result in significantly potentiated activity against coccidiosis caused by Eimeria that have developed resistance to this anticoccidial. The activity of the present compositions is greater than would be expected from a simple additive effect of the frenolicin B and the potentiated sulfonamide components, i.e. is synergistic.

Accordingly, the present invention is directed to anticoccidial compositions, i.e compositions which are useful for combating, i.e preventing and treating, coccidiosis in animals, comprising a mixture of frenolicin B, sulfadimethoxine and ormetoprim, wherein these ingredients are present in amounts which in combination are synergistically effective in combating at least one coccidiosis-causing strain of Eimeria.

These compositions are especially useful by virtue of their potentiated activity against strains of Eimeria which have become resistant to the aforementioned chemical anticoccidial which has been approved for and used in the treatment of coccidiosis, especially in poultry.

The compositions of the present invention, when administered to animals such as domestic animals and animals raised commercially for food, such as poultry, cattle, sheep and pigs, are effective in the prophylaxis and treatment of coccidiosis. The frenolicin B synergistically interacts with the sulfonamide component (sulfadimethoxine) of the present compositions resulting in greater activity against resistant coccidiosis. Another advantage of the present invention is that the use of the frenolicin B makes possible the use of reduced amounts of the active ingredients with resulting reduction in the risk of toxicity and the side effects typically associated therewith, such as adverse influence on feeding, water intake and nutrient absorption.

The production and isolation from Streptomyces roseofulvus of the frenolicin B utilized in the present invention is disclosed in the article by Iwai et al. in J. Antibiotics, Vol. 31, No. 10, pp. 959-965 (1978).

The sulfonamides are a known class of antibiotics which are characterized by the radical SO₂NH₂. Examples of such sulfonamides are those described in U.S. Patent Specification No. 3,461,206. In the compositions of the present invention sulfadimethoxine [4-amino-N-(2,6-dimethoxy)-4-pyrimidinyl)-benzenesulfonamide] is used as the sulfonamide component.

This sulfonamide is employed in the present compositions in conjunction with a potentiator of its activity, namely the substituted pyrimidine known as ormetoprim. This compound belongs to the class of 2,4-diamino-5-(trisubstituted benzyl)-pyrimidine sulfonamide potentiators of the formula: 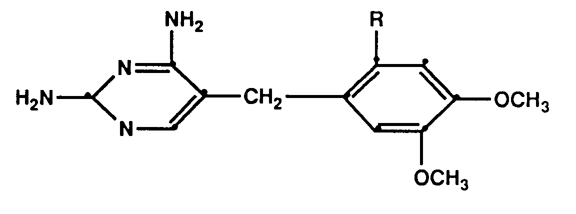
in which R is methoxy, methyl or ethyl.

Exemplary of such sulfonamide potentiators of the above formula are the following substituted pyrimidines:
2,4-diamino-5-(2',4',5'-trimethoxybenzyl)-pyrimidine(trimethoprim),
2,4-diamino-5-(2'-methyl-4',5'-dimethoxybenzyl)-pyrimidine (ormetoprim) and
2,4-diamino-5-(2'-ethyl-4',5'-dimethoxybenzyl)-pyrimidine
As indicated above the potentiated sulfonamide used in the present compositions is the combination of sulfadimethoxine and ormetoprim.

The potentiator ormetoprim is generally employed in these compositions in amounts ranging from about 0.01 to about 20 parts by weight for each part by weight of the sulfonamide sulfadimethoxine, the weight ratio of sulfadimethoxine to ormetoprim thus being in the range from about 100:1 to about 0.05:1. More effectively, however, the weight ratio of the sulfadimethoxine to ormetoprim in the compositions is in the range from about 20:1 to about 1:1, with especially efficacious results being obtained using weight ratios of about 5:1 or particularly about 5:3.

The individual components of the compositions of the present invention are employed in relative amounts which are synergistic in combating coccidiosis-producing microorganisms and in particular against those strains of Eimeria which have developed resistance to the sulfonamide anticoccidials due to exposure over time to the latter. A preferred weight ratio range of frenolicin B to the potentiated sulfonamide component, i.e. one which results in particularly good synergistic effects, is from about 2:9 to about 1:1.

The present invention further embraces improved anticoccidial animal comestible and drinking water compositions. Such anticoccidial compositions comprise an animal feed or drinking water, as appropriate, in admixture with (or containing) an anticoccidial composition as defined hereinabove, wherein the combination of sulfadimethoxine and ormetoprim is present in an amount from about 75 to about 150 ppm by weight of the animal feed or drinking water component, and the frenolicin B is present in an amount which combined with the amount of sulfadimethoxine and ormetoprim is synergistically effective in combating at least one coccidiosis-causing strain of Eimeria. These anticoccidial animal comestible compositions may be prepared by mixing the active ingredients, i.e. frenolicin B, sulfadimethoxine and ormetoprim, with suitable carrier or diluent material generally used as animal feed or with drinking water, as appropriate.

The concentration of the active ingredients in the anticoccidial animal comestible or drinking water compositions of this invention can be adjusted to meet particular needs and may vary over a wide range. The limiting criteria of the concentration are that the minimum concentration is such that a sufficient amount of the active ingredients is provided to effect the desired control of coccidiosis and the maximum concentration is such that the amount of composition ingested does not result in any untoward or undesirable side effects. This may, of course, vary according to the potency and usual recommended dosing level for the involved anticoccidial agents Furthermore, because of the potentiating interaction of the active ingredients of the present invention, the amounts of each component required may usefully be less than that which would ordinarily be considered the usual recommended dosing level for the individual components if used separately as a coccidiostat. The amount of sulfadimethoxine necessary for successfully combating coccidiosis may therefore be beneficially reduced. Thus, the potentiated sulfonamide component is typically present in an amount of from about 25 to about 90%, and preferably 33% to 75%, of the normal recommended dosing level for that anticoccidial agent. For example, the approved recommended dosing level for the combination of sulfadimethoxine and ormetoprim for the prevention of coccidiosis in chickens would be about 200 ppm (125 ppm of sulfadimethoxine and 75 ppm of ormetoprim) based on the weight of the animal feed or drinking water component. However, the compositions in accordance with the present invention preferably contain about 33 to 75 ppm of frenolicin B, the combination of sulfadimethoxine and ormetoprim being present in an amount of from about 75 to about 150 ppm, the amounts in ppm being by weight relative to the weight of the animal feed or drinking water component.

The easiest way to administer the anticoccidial compositions of the present invention is by providing the animals to be treated with an anticoccidial animal comestible or drinking water composition of the present invention. However, the anticoccidial compositions can be usefully administered in other ways. For example, they can be incorporated into tablets, drenches, boluses, or capsules, and dosed to the animals. Formulations of the antibiotic compounds in such dosage forms can be accomplished by means of methods well-known in the veterinary pharmaceutical art. Of course, whatever the method or methods of administration, the two components of the composition of the present invention can be administered independently so long as the synergistically effective combination is ultimately dosed to the animal. All these methods of administration represent further aspects of the present invention.

As mentioned hereinbefore, the most practical way to treat animals with the anticoccidial compositions is by the providing the animals with the above-defined anticoccidial comestible or drinking water compositions. Any type of feed may be medicated with the anticoccidial compositions, including common dry feeds, liquid feeds and pelleted feeds.

The methods of formulating anticoccidials into animal feeds are well-known. It is usual to make a concentrated anticoccidial premix as a raw material for medicated, in the present case anticoccidial, feeds. For example, typical anticoccidial premixes may contain from about 2.0 to about 150 grams of anticoccidial per pound of premix (approx. 4.4 g/kg - 331 g/kg). The wide range results from the wide range of concentration of anticoccidial which may be desired in the final feed. The animal feed additive premix embraced by the present invention comprises about 4.4 g/kg to about 331 g/kg of an anticoccidial composition, said composition consisting of a mixture of frenolicin B, sulfadimethoxine and ormetoprim present in amounts which in combination are synergistically effective in combating at least one coccidiosis-causing strain of Eimeria. These premixes may be either liquid or dry formulations, and contain, apart from the aforementioned active ingredients, conventional edible carrier material suitable for the animals concerned. As in the case of the anticoccidial compositions, animal comestible compositions and animal drinking water compositions of the present invention the weight ratio of sulfadimethoxine to ormetoprim is generally in the range from about 100:1 to about 0.05:1, preferably, about 20:1 to about 1:1, and the preferred weight ration range of frenolicin B to the potentiated sulfonamide component is from about 2:9 to about 1:1.

The formulation of animal feeds containing the proper amounts of anticoccidial composition for useful treatment can be effected in conventional manner. It is necessary only to calculate the amount of composition which it is desired to administer to the animals in question, taking into account the amount of feed per day which the animals eat and the concentration of anticoccidial composition (mixture of the active ingredients) as such or in the premix to be used, and calculate the appropriate concentration of anticoccidial composition or of premix in the feed and thus the dilution factor.

The known methods of formulating, mixing and processing feeds which are normally used in the animal feed arts are entirely appropriate for manufacturing feeds containing the anticoccidial compositions of the present invention. For example, separately formulated premixes for each of the two components (frenolicin B and the mixture of sulfadimethoxine and ormetoprim) could be added to a given feed lot to provide an anticoccidial animal comestible composition of the present invention. Analogous considerations apply for the formulation of the anticoccidial drinking water compositions of the present invention.

The compositions of the present invention are typically effective in combating coccidiosis when administered to animals in feed mixes containing the compositions (mixture of the active ingredients) in a concentration of from about 100 ppm to about 250 ppm by weight.

The following examples are illustrative of the invention.

### Example 1

### Anticoccidial Activity against Sulfadimethoxine/Ormetoprim-Resistant Field Strain of E. tenella

### Experimental Procedure

Two-week old Arbor Acres, Peterson Cross broiler chickens, obtained from a commercial hatchery and kept in wire-floored, electrically heated, battery brooders, were used in all studies. Male birds, selected according to weight, were used in each group. The chickens were medicated two days before infection and maintained on the medicated feed until the termination of the trial for a total medication period of nine days. There were two replicates of eight birds each per treatment.

### Eimeria Field Isolates

The E. tenella field isolate was recovered from a commercial broiler operation in North Carolina. The intestinal samples were homogenized with a Waring Blender, suspended in 2.5% potassium dichromate solution, sporulated at room temperature, and inoculated into young susceptible, coccidia-free chicks for further oocyst collection and species identification.

At appropriate post-infection times, 5 to 7 days, the birds were sacrificed, autopsied, and diagnosis of Eimeria species made, based on location site, and oocysts measurement. Oocysts were collected from the intestines of the passaged birds and sporulated.

In evaluating the efficacy of the frenolicin B combinations, 3.0 x 10⁵ sporulated oocysts were administered per bird. The sporulated oocysts, properly agitated and suspended in sterile distilled water, were inoculated in volumes of 1.0 ml directly into the chick crop by means of a blunt needle attached to a calibrated syringe.

### Criteria for Evaluating Anticoccidial Efficacy

At termination of the trials, the surviving birds were sacrificed, necropsied and scored for gross lesions. All birds that died during the experiments were also necropsied; proper diagnosis was made, and their gross lesions scored and recorded. The intestinal lesions were scored as 0 = normal, 1 = slight, 2 = moderate, 3 = severe and 4 = dead. The readings obtained were summarized as average degree of infection (ADI).

In addition, bird group weight, feed intake, feed conversion and mortality were recorded.

### Results

The results are set forth in Table 1 below:

**Table 1**

| Anticoccidial Activity of Frenolicin B in Combination with Sulfadimethoxine/Ormetoprim (5:3) against Eimeria tenella | | | | | |
|---|---|---|---|---|---|
| Treatment | Level (ppm) | Weight Gain, % | Feed Conversion | Lesion Score | % Mortality |
| UUC¹ | 0 | 100 | 1.60 | 0.0 | 0 |
| IUC² | 0 | 74 | 2.36 | 2.7 | 18.75 |
| Frenolicin B | 80 | 89 | 1.80 | 1.0 | 0 |
| Frenolicin B | 40 | 89 | 1.95 | 1.6 | 0 |
| Sulfadimethoxine/ormetoprim (5:3) | 200 | 86 | 1.76 | 1.6 | 0 |
| Sulfadimethoxine/ormetoprim (5:3) | 100 | 63 | 3.31 | 3.0 | 25.0 |
| Sulfadimethoxine/ormetoprim (5:3) + Frenolicin B | 200+80 | 99 | 1.80 | 0.2 | 0 |
| Sulfadimethoxine/ormetoprim (5:3) + Frenolicin B | 200+40 | 98 | 1.59 | 0.5 | 0 |
| Sulfadimethoxine/ormetoprim (5:3) + Frenolicin B | 100+80 | 96 | 1.71 | 0.8 | 0 |
| Sulfadimethoxine/ormetoprim (5:3) + Frenolicin B | 100+40 | 94 | 1.97 | 1.0 | 0 |

| | | | | | |
|---|---|---|---|---|---|
| ¹ UUC = uninfected unmedicated chickens | | | | | |
| ² IUC = infected unmedicated chickens | | | | | |

### Example 2

A typical animal feed additive premix contains 22.7 g frenolicin B/lb premix (approx. 50.05 g/kg), 113,5 g sulfadimethoxine/lb premix (approx. 250.25 g/kg) and 68.1 g ormetoprim/lb premix (approx. 150.15g/kg), the rest being made up of rice bran, rice hulls, soybean or mineral oil and optionally other edible carrier materials(s). The nature and relative contents of the edible carrier materials can be varied at will as appropriate for the type of animals for which the anticoccidial comestible composition, made up from the premix, is ultimately intended.

Such and other animal feed additive premixes can be added, e.g. at the rate of 1 lb (0.454 kg)/ton, to an animal feed, e.g. of the following compositions, to produce typical anticoccidial animal comestible compositions for very young ("starter"), growing ("grower") and fully grown ("finisher") poultry (chickens, hens):

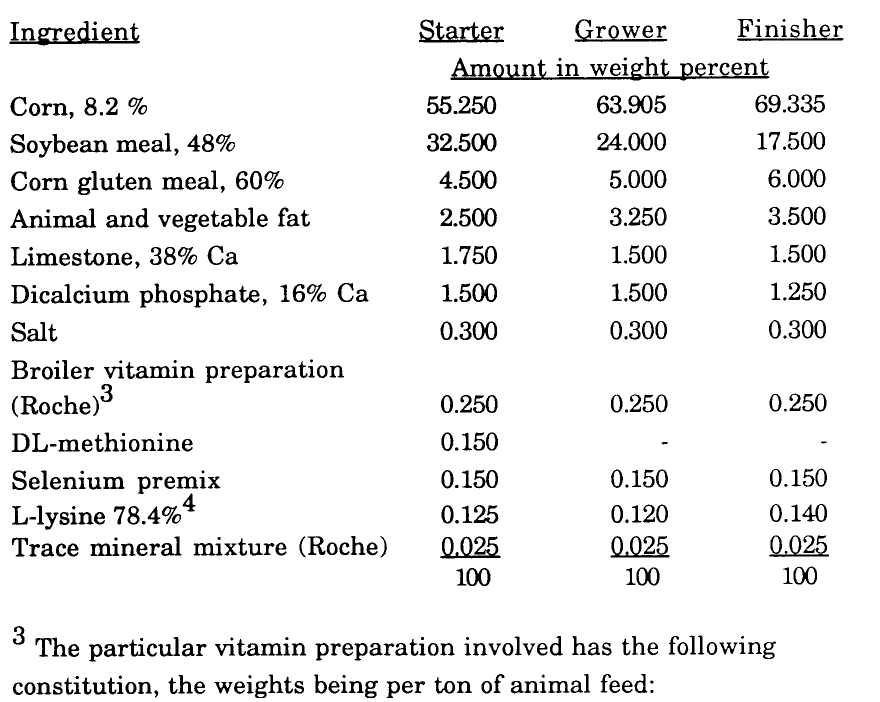

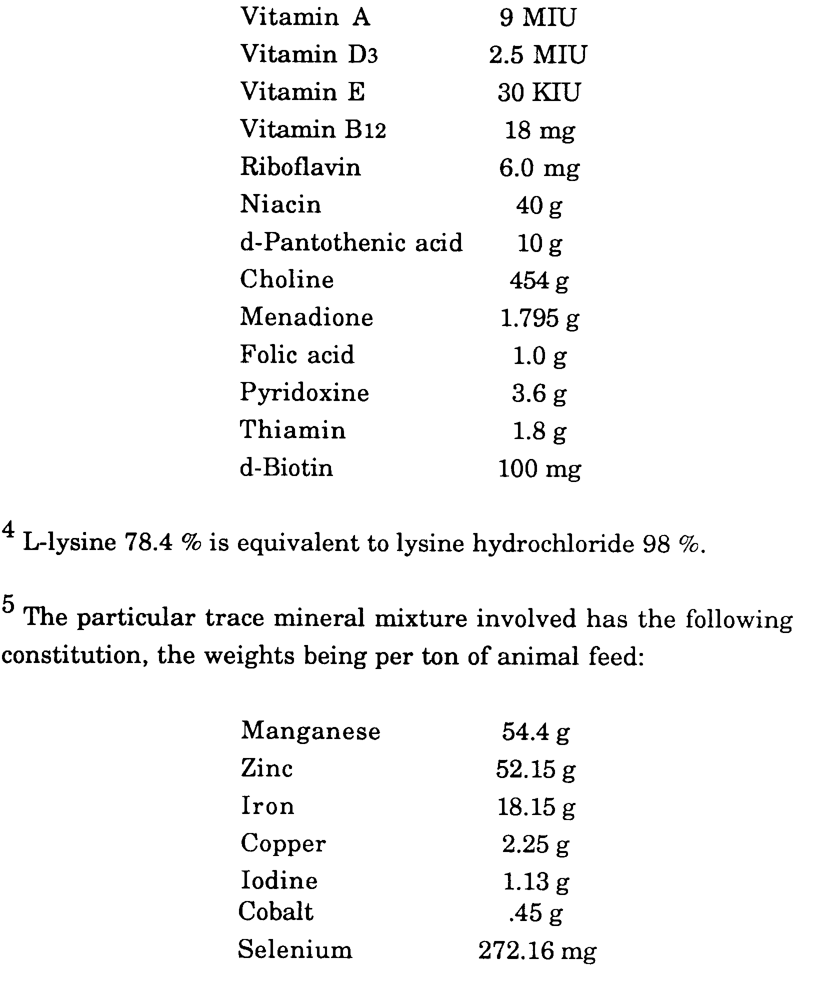

The so obtained anticoccidial animal comestible composition contains final active ingredient concentrations of 30 ppm frenolicin B and 125 ppm sulfadimethoxine/ormetoprim.

## Claims

1. A composition for combating coccidiosis in animals comprising a mixture of frenolicin B, sulfadimethoxine and ormetoprim, wherein these ingredients are present in amounts which in combination are synergistically effective in combating at least one coccidiosis-causing strain of Eimeria.

2. A composition according to claim 1, wherein the weight ratio of sulfadimethoxine to ormetoprim is in the range from about 100:1 to about 0.05:1.

3. A composition according to claim 2, wherein the weight ratio is in the range from about 20:1 to about 1:1.

4. A composition according to any preceding claim, wherein the weight ratio of frenolicin B to the combination of sulfadimethoxine and ormetoprim is in the range from about 2:9 to about 1:1.

5. An anticoccidial animal comestible composition comprising an animal feed in admixture with a composition for combating coccidiosis in animals, said latter composition comprising a mixture of frenolicin B, sulfadimethoxine and ormetoprim, wherein the combination of sulfadimethoxine and ormetoprim is present in an amount from about 75 to about 150 ppm by weight of the animal feed component and the frenolicin B is present in an amount which combined with the amount of sulfadimethoxine and ormetoprim is synergistically effective in combating at least one coccidiosis-causing strain of Eimeria.

6. A composition according to claim 5, wherein the amount of frenolicin B is from about 33 to about 75 ppm by weight of the animal feed, and the combination of sulfadimethoxine and ormetoprim is present in an amount of from about 75 to about 150 ppm by weight of the animal feed.

7. An anticoccidial animal drinking water composition comprising drinking water containing a composition for combating coccidiosis in animals, said latter composition comprising a mixture of frenolicin B, sulfadimethoxine and ormetoprim, wherein the combination of sulfadimethoxine and ormetoprim is present in an amount from about 75 to about 150 ppm by weight of the drinking water component and the frenolicin B is present in an amount which combined with the amount of sulfadimethoxine and ormetoprim is synergistically effective in combating at least one coccidiosis-causing strain of Eimeria.

8. An animal feed additive premix comprising about 4.4 g/kg to about 331 g/kg of an anticoccidial composition, said composition consisting of a mixture of frenolicin B, sulfadimethoxine and ormetoprim present in amounts which in combination are synergistically effective in combating at least one coccidiosis-causing strain of Eimeria.

9. An animal feed additive premix according to claim 8, wherein the weight ratio of sulfadimethoxine to ormetoprim is in the range from about 100:1 to about 0.05:1, and the weight ratio of frenolicin B to the combination of sulfadimethoxine and ormetoprim in the range from about 2:9 to about 1:1.

## Claims (Claims for the following Contracting State(s): ES)

1. A process for producing a composition for combating coccidiosis in animals comprising admixing frenolicin B, sulfadimethoxine and ormetoprim in amounts which in combination are synergistically effective in combating at least one coccidiosis-causing strain of Eimeria.

2. A process according to claim 1, wherein the weight ratio of sulfadimethoxine to ormetoprim is in the range from about 100:1 to about 0.05:1.

3. A process according to claim 2, wherein the weight ratio is in the range from about 20:1 to about 1:1.

4. A process according to any preceding claim, wherein the weight ratio of frenolicin B to the combination of sulfadimethoxine and ormetoprim is in the range from about 2:9 to about 1:1.

5. A process for producing an anticoccidial animal comestible composition comprising admixing an animal feed with a composition for combating coccidiosis in animals, said latter composition comprising a mixture of frenolicin B, sulfadimethoxine and ormetoprim, wherein the combination of sulfadimethoxine and ormetoprim is present in an amount from about 75 to about 150 ppm by weight of the animal feed component and the frenolicin B is present in an amount which combined with the amount of sulfadimethoxine and ormetoprim is synergistically effective in combating at least one coccidiosis-causing strain of Eimeria.

6. A process according to claim 5, wherein the amount of frenolicin B is from about 33 to about 75 ppm by weight of the animal feed, and the combination of sulfadimethoxine and ormetoprim is present in an amount of from about 75 to about 150 ppm by weight of the animal feed.

7. A process for producing an anticoccidial animal drinking water composition comprising admixing drinking water with a composition for combating coccidiosis in animals, said latter composition comprising a mixture of frenolicin B, sulfadimethoxine and ormetoprim, wherein the combination of sulfadimethoxine and ormetoprim is present in an amount from about 75 to about 150 ppm by weight of the drinking water component and the frenolicin B is present in an amount which combined with the amount of sulfadimethoxine and ormetoprim is synergistically effective in combating at least one coccidiosis-causing strain of Eimeria.

8. A process for producing animal feed additive premix comprising admixing edible carrier material with about 4.4 g/kg to about 331 g/kg of an anticoccidial composition, said composition consisting of a mixture of frenolicin B, sulfadimethoxine and ormetoprim present in amounts which in combination are synergistically effective in combating at least one coccidiosis-causing strain of Eimeria.

9. A process according to claim 8, wherein the weight ratio of sulfadimethoxine to ormetoprim is in the range from about 100:1 to about 0.05:1, and the weight ratio of frenolicin B to the combination of sulfadimethoxine and ormetoprim in the range from about 2:9 to about 1:1.
